# EUROPÄISCHE PATENTSCHRIFT

(11) **EP 1 154 837 B1**
(45) Veröffentlichungstag und Bekanntmachung des Hinweises auf die Patenterteilung: **07.04.2004**
(21) Anmeldenummer: 00916744.6
(22) Anmeldetag: 11.02.2000
(51) Int. Cl.: B01D 59/44, G01N 1/28

(54) **VERFAHREN UND VORRICHTUNG ZUR FREISETZUNG VON SAUERSTOFFISOTOPEN AUS SAUERSTOFFHALTIGEN FESTSTOFFEN**
METHOD AND DEVICE FOR LIBERATING OXYGEN ISOTOPES FROM OXYGEN-CONTAINING SOLIDS
PROCEDE ET DISPOSITIF POUR LIBERER DES ISOTOPES D'OXYGENE DE SOLIDES CONTENANT DE L'OXYGENE

(30) Priorität: 18.02.1999 DE 19906732
(43) Veröffentlichungstag der Anmeldung: 21.11.2001
(73) Patentinhaber: Forschungszentrum Jülich GmbH, 52425 Jülich (DE)
(72) Erfinder: SCHLESER, Gerhard, Hans, D-41238 Mönchengladbach (DE); KNÖRCHEN, Wolfgang, D-52457 Aldenhoven (DE)
(86) Internationale Anmeldenummer: PCT/DE2000/000425
(87) Internationale Veröffentlichungsnummer: WO 2000/049623

(56) Entgegenhaltungen:
- FR-A- 2 734 363
- GB-A- 2 184 235

## Beschreibung

Die Erfindung betrifft ein Verfahren zur Freisetzung von Sauerstoffisotopen aus Silikaten, insbesondere aus biogenen und abiogenen silikatischen Substanzen beziehungsweise Feststoffen sowie eine für die Durchführung des Verfahrens geeignete Vorrichtung.

In der Paläoklimatologie und der Paläothermometrie besteht Interesse an der Feststellung der in Feststoffproben vorhandenen Verhältnisse der Sauerstoffisotope ¹⁶O und ¹⁸O.
Aus dem Werk "Stable Isotope Geochemistry" von J.Hoefs 4^{th} Completely Revised, Ubdated and Enlaged Edition; Springer Berlin, Heidelberg, New York, Barcelona, Budapest, Hong Kong 1997, sind verschiedene Methoden zur Freisetzung von Sauerstoff aus Proben bekannt. So werden nach dem Stand der Technik zu untersuchende silikatische Proben von Feststoffen durch Laserbestrahlung verdampft, wobei Sauerstoff freigesetzt und der Analyse zugänglich gemacht wird. Der Sauerstoff kann und wird vielfach direkt massenspektroskopisch auf seine Isotope untersucht. Alternativ kann der Sauerstoff in einer weiteren Reaktion mit Kohlenstoff in CO oder CO₂ überführt werden. Die Ermittlung des Isotopenverhältnisses erfolgt dann durch massenspektroskopische Untersuchung des durch Reaktion mit Graphit entstandenen CO oder CO₂.
Ein weiteres, bei Silikaten häufig zum Einsatz kommendes Verfahren, basiert auf der Fluorinierung der Substanzen, deren Sauerstoffisotope analysiert werden sollen. Dabei wird der Sauerstoff mittels F₂ oder BrF₅ in Nickelzylindern bei 500-600 °C freigesetzt. Der Sauerstoff wird danach im allgemeinen an heißem Graphit in CO₂ überführt und anschließend der massenspekroskopischen Messung zugeführt. Vielfach erfordern die zu untersuchenden Proben eine Vorreinigung, weil die Analyse störende Fremdmoleküle oder Gruppen in die Probe eingelagert sind. So enthalten z.B. biogene Silikate, wie Schalen von Diatomeen, vielfach Wasser oder OH-Gruppen, die eine analytische Sauerstoffisotopenbestimmung stark erschweren. Soll daher eine Analyse betreffend der Isotopenzusammensetzung des Sauerstoffes vorgenommen werden, so muß die Probe zunächst vielfach in vorgeschalteten Reinigungsschritten von Fremdstoffen beziehungsweise Molekülen befreit werden, welche das Analysenergebnis verfälschen können. Eine Methode zur Freisetzung von Sauerstoff aus den zu untersuchenden Proben ist die Laserverdampfung partieller Bereiche von Festkörpern. Bei dieser Methode ist eine, wenn auch geringe Fraktionierung, das heißt eine Verschiebung des Sauerstoffisotopenverhältnisses der zu analysierenden Probe des Festkörpers, nicht zu vermeiden, die durch die etwas anderen Bedingungen in den Randbereichen des Verdampfungsvorgangs begründet sind. Die Erhitzung der Feststoffprobe durch Laser hat darüber hinaus im Hinblick auf die Reproduzierbarkeit beziehungsweise die Repräsentativität des Ergebnisses von größeren Proben erhebliche Nachteile, denn mit dem Laserstrahl werden lediglich punktuelle Bereiche der gesamten Feststoffprobe erhitzt und damit der Isotopenanalyse zugänglich gemacht. Bei Inhomogenitäten einer Probe ist somit das Ergebnis der zugehörigen Messung des Verhältnisses der Sauerstoffisotope nicht unbedingt repräsentativ für die gesamte Probe. Um repräsentative Ergebnisse zu erlangen sind daher mehrere Messungen notwendig, deren Ergebnisse gemittelt werden müssen.

Die FR-A-2 734 363 (PERNOD RICARD) 22. November 1996 (1996-11-22) offenbart ein Verfahren und eine Vorrichtung zur Messung des Gehaltes ¹⁸O und/oder¹⁵N in einer chemischen Verbindung, bei denen die Substanz in Anwesenheit von Kohlenstoff pyrolysiert und anschließend analysiert wird.

In der GB-A-2 184 235 (DORYOKURO KAKUNENRYO) 17. Juni 1987 (1987-06-17) wird ein Verfahren zur Bestimmung des Sauerstoff/Metallverhältnisses in nuclearem Brennmaterial beschrieben. Der Sauerstoff des Brennmaterials wird durch Reaktion des Kohlenstoffs des Graphit-Tiegels in CO umgewandelt, wonach das Verhältnis Sauerstoff zu Metall bestimmt wird.

Die GB 1052907 A betrifft eine Vorrichtung zur Veredelung von Kupfer und dessen Legierungen, bei der das Kupfer durch Induktion und in Kontakt bringen mit Graphit veredelt wird.

Es ist daher die Aufgabe der Erfindung, ein Verfahren und eine Vorrichtung zu schaffen, mit denen silikatische Feststoffproben in einem Arbeitsgang ohne Vorbehandlung, etwa zur Freisetzung an der Probe anhaftender H₂O-Moleküle oder OH-Gruppen, analysiert werden können, auch ohne Mittelwertbildung der Ergebnisse vornehmen zu müssen.

Ausgehend vom Oberbegriff des Anspruchs 1 wird die Aufgabe erfindungsgemäß gelöst mit den im kennzeichnenden Teil des Anspruchs 1 angegebenen Merkmalen.

Mit dem erfindungsgemäßen Verfahren ist es nunmehr möglich, Ergebnisse für die Verhältnisse der Isotopenzusammensetzung des Sauerstoffes in Feststoffproben von so problematischem Material, wie biogene Silikate, in einem experimentellen, sequentiell aufgebauten Arbeitsschritt zusammenzufassen. Für die Probenvorpräparation ist damit kein gesonderter experimenteller Schritt mehr notwendig. Damit ist eine erhebliche Reduzierung des Arbeits- und Zeitaufwandes möglich. Weiterhin ermöglicht das erfindungsgemäße Verfahren die Analyse des Sauerstoffisotopenverhältnisses von Proben, ohne daß sich Inhomogenitäten der Probe auf die Qualität des Meßergebnisses im Hinblick auf die Gesamtprobe auswirken. Neben Mengen, die massensprektroskopisch problemlos meßbar sind, kann zum Beispiel für Silikate die Probenmenge im Flowmode (Fließmodus) auf 50 µg SiO₂ reduziert werden. Möglich sind aber auch geringere Einwaagen: wie 25 µg SiO₂, die mit chemischen Methoden des Aufschlusses so gut wie nicht handhabbar sind. Bei sehr heterogenen Proben werden jedoch größere Proben von wenigstens 100 µg SiO₂ oder mehr bevorzugt, um einen verläßlichen Mittelwert zu erlangen.

Vorteilhafte Weiterbildungen der Erfindung sind in den Unteransprüchen angegeben.

Die Zeichnung zeigt eine Darstellung der erfindungsgemäßen Vorrichtung in schematischer Form.

Es zeigt:

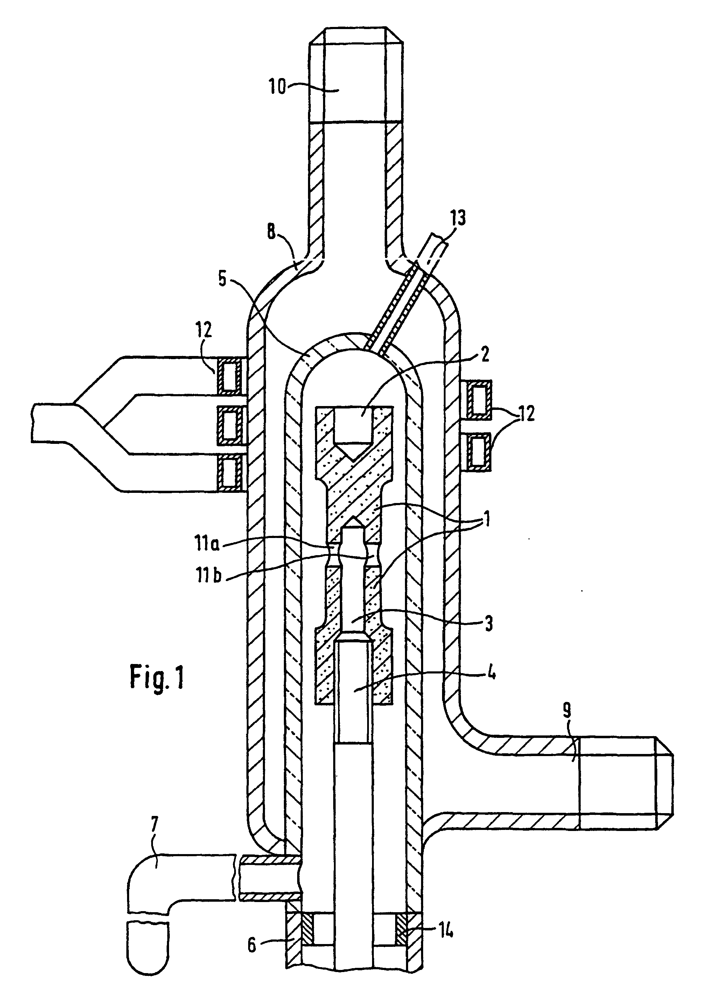

Fig.1: Eine erfindungsgemäße Vorrichtung.

Die in Figur 1 dargestellte erfindungsgemäße Vorrichtung beinhaltet eine Graphitküvette 1, welche längsförmig ausgebildet ist, wobei sich am oberen Ende eine Aushöhlung 2 zur Aufnahme eines Feststoffes befindet, und am entgegengesetzten Ende eine Bohrung 3 vorhanden ist, welche einen Stab 4 aufnehmen kann, mit dem die Graphitküvette 1 in ein Gehäuse 5 eingebracht werden kann, welches aus Quarzglas besteht. Das Gehäuse 5 ist über ein Verbindungsteil 6 vakuumdicht an eine Pumpe anschließbar, welche in der Figur 1 nicht dargestellt ist. Für spezielle Untersuchungen ist eine Kühlfalle 7 integriert, in der etwa thermisch aus der Probe ausgetriebenes Wasser aufgefangen werden kann. Das Gehäuse 5, welches die Graphitküvette 1 beinhaltet, ist von einem Kühlmantel 8 umschlossen, welcher einen Kühlmitteleingang 9 und einen Kühlmittelausgang 10 aufweist. Die Graphitküvette 1 besitzt Bohrungen 11a, b, welche bezüglich der Längsachse der Graphitküvette 1 radial verlaufen und eine Verbindung zwischen der Bohrung 3 und der Umgebung der Graphitküvette 1 herstellen. Das Gehäuse 5 ist in der Höhe der Aushöhlung 2 der Graphitküvette 1 von einer Induktionsspule 12 umgeben, welche mit einem Mittelfrequenz-Generator (MF-Generator) in Verbindung steht. In den Kühlmantel 8 sind an der Außenseite im Winkel von 180° zwei Heliumleitungen 13 integriert, durch die das Trägergas Helium in das Gehäuse 5 einströmen kann, welches als Trägermaterial für CO oder CO₂ dient, das einem Massenspektrometer zugeführt wird. Im Verbindungsteil 6 befindet sich ein Palladiumring 14.

Bei Betrieb der erfindungsgemäßen Vorrichtung wird die Graphitküvette 1 mit der Feststoffprobe zunächst mit dem Stab 4 in das Gehäuse 5 manuell oder automatisch eingebracht. An einem Adapter, welcher an das Verbindungsteil 6 angeschlossen ist, wird die Pumpe zugeschaltet. Das Gehäuse 5 besteht aus Quarzglas, da dieses für die experimentellen Bedingungen inert ist und kein Austausch bei den hohen Temperaturen der Sauerstoffatome des Quarzglases mit dem im Experiment entstehenden Sauerstoff stattfindet. Der Kühlmantel 8 wird über den Kühlmitteleingang 9 von Wasser durchströmt, welches durch den Kühlmittelausgang 10 abgeführt wird. Die Wasserkühlung liegt im Beipaß des MF-Generatorkühlkreislaufs, durch den über einen weiteren Beipaß auch die Induktionnsspule gekühlt wird. Die Induktionsspule wird über den MF-Generator eingeschaltet, so daß sich die Graphitküvette 1 induktiv erhitzt. Die Aufheizzeit ist frei wählbar und stufenlos bis 2200°C regelbar. Die Aufheizung kann jedoch, wenn erforderlich, in Stufen erfolgen. Die gewünschten Temperaturen lassen sich entweder manuell oder über eine im Netzteil integrierte Schnittstelle programmierbar einstellen. Erfindungsgemäß kann die Silikatprobe ohne vorherige Aufreinigung, das heißt zum Beispiel durch Desorption von Wasser oder OH⁻-Gruppen in der Graphitküvette 1 der Induktionsheizung ausgesetzt werden. Hierbei erhitzt sich die Graphitküvette 1, was zu einer gleichzeitigen Aufheizung der Silikatprobe führt. Die Temperatur kann nun langsam gesteigert werden, wobei die Aufheizung manuell oder programmierbar erfolgen kann. Bei einer Temperatur von ca. 100-120 °C kann Wasser desorbiert werden, welches durch die Pumpe abgesaugt wird. Durch weitere Temperatursteigerung können weitere Verunreinigungen fraktioniert abdampfen. So werden bei einer Temperatur von ca. 1000°C alle eingelagerten OH⁻-Gruppen abgetrennt. Weiterhin können stickstoffhaltige Verbindungen ausgetrieben werden, welche die spätere Analyse aufgrund des Molekulargewichtes von N₂ quantitativ beeinträchtigen und verfälschen können. Bei Temperaturen von ca. 1300-1400°C sind die Verunreinigungen quantitativ abgetrennt und eine weitere Steigerung der Temperatur führt sukzessive dazu, daß Silikat Sauerstoff abspaltet, welcher mit dem Graphit der Graphitküvette 1 zu CO reagiert. Die Aufheizung kann problemlos bis zu einer Temperatur von 2200 °C durchgeführt werden. Bei diesen experimentellen Bedingungen liegt ein Bouduard-Gleichgewicht zwischen CO und CO₂ vor, welches ganz auf der Seite des CO liegt. Das Vorliegen von CO hat gegenüber CO₂ den Vorteil, daß das CO, als zu analysierender Stoff, zu einer doppelt so hohen Empfindlichkeit bezüglich der Massenspektrometrie führt als CO₂. Weiterhin ist das Experiment mit CO kürzer, so daß Arbeitszeit gespart wird. Daher wird bevorzugt eine induktive Erhitzung auf hohe Temperaturen vorgenommen, bei denen das Bouduard-Gleichgewicht auf der Seite des CO liegt. Alternativ kann das durch die Reaktion des Sauerstoffes mit dem Graphit entstehende CO auch durch Temperaturabsenkung in der Graphitküvette 1 aufgrund des Bouduard-Gleichgewichtes in CO₂ überführt werden. Liegt das Gleichgewicht auf der Seite des CO₂, so kann dieses in der Kühlfalle 7 auskondensiert werden. Im allgemeinen, vor allem im Routinebetrieb, wird das CO₂ jedoch im Helium-Trägergasstrom, welcher durch die Heliumleitung 13 eingeführt wird, zum Massenspekrometer transportiert. Aufgrund der verschiedenen Atomgewichte der beiden Sauerstoffisotope kann das entstehende CO ein Molekulargewicht von 28 oder 30 und das damit im Gleichgewicht stehende CO₂ ein Molekulargewicht von 44 oder 46 haben. Daher kann es auch vorteilhaft sein, bei einer Temperatur zu arbeiten, bei der das Bouduard-Gleichgewicht auf der Seite des CO₂ liegt, da dessen Molekulargewicht nicht mit dem von N₂ zu Überlagerungen in den entsprechenden Faraday Auffängern bei massenspektroskopischen Messungen führen kann.
Das Verfahren ist bei allen Sauerstoff enthaltenden Feststoffen anwendbar, unabhängig davon, ob sie in Pulverform, amorpher Masse, als Kristall oder sonstiger fester Form vorliegen.
Die Möglichkeit der kontinuierlichen Aufheizung von Probenmaterial gestattet die Vorreinigung der Substanzen von unerwünschten Fremdstoffen bzw. Molekülen durch fraktionierte Desorption oder Abspaltung. Hierbei wird eine Verfälschung des Ausgangsverhältnisses der Sauerstoffisotope weitgehend verhindert, da im Gegensatz zur chemischen Aufarbeitung kein Austausch der Sauerstoffatome der Feststoffprobe mit Sauerstoffatomen von Reagenzien stattfindet.
Bei Gemischen, die unterschiedliche Zersetzungstemperaturen bzw. Schmelz- oder Verdampfungstemperaturen besitzen, kann eine sequentielle Sauerstoffisotopenbestimmung der unterschiedlichen Komponenten des Gemisches durchgeführt werden.
In einer alternativen Ausführungsform des erfindungsgemäßen Verfahrens wird die zu analysierende bzw. freizusetzende Probe an zu untersuchendem Feststoff mit Graphitpulver vermischt und dieses Gemisch induktiv erhitzt. Das Gemisch kann gegebenenfalls festgepreßt werden.
Die in der erfindungsgemäßen Vorrichtung eingesetzte Graphitküvette 1 besitzt Bohrungen 11a,b, welche eine Verbindung zwischen der Bohrung 3 zur Aufnahme des Stabes 4 zum Einbringen der Graphitküvette 1 in das Gehäuse 5 und dem Innenraum des Gehäuses 5 herstellen. Dies ist dann von Vorteil, wenn sich in der Bohrung 3 Verunreinigungspartikel abgesetzt haben, welche die Analyse stören können. Die Bohrungen 11a,b begünstigen eine Ablösung von Fremdpartikeln, weil diese einen leichteren Ausgang aus der Bohrung 3 finden.
Grundsätzlich kann die Graphitküvette 1 auch jede andere Geometrie besitzen, so daß die Ausbildung der Graphitküvette 1 nicht auf die in Figur 1 dargestellte Ausgestaltung beschränkt ist. Ein kreisrunder Querschnitt ist jedoch für die induktive Einkopplung, die Küvettenmanipulation und die Küvettenherstellung am günstigsten.
Der Graphit ist für die analytischen Zwecke hochrein so daß keine Verfälschung der Analysenergebnisse durch Verunreinigungen stattfinden kann. Bevorzugt ist die Verwendung von Spektralgraphit, der Zusatzbestandteile lediglich in einer Konzentration von unter 20 ppm mit Si <2ppm, Ca <1ppm, B <1ppm, Fe <0,5ppm, Ti <0,5ppm aufweist. Je nach den nachgeschalteten Analysenverfahren, welche nicht auf Massenspekrometrie beschränkt sind, kann die geforderte Reinheit jedoch auch nach unteren Werten abweichen.
Mit dem erfindungsgemäßen Verfahren können größere Feststoffmengen von beispielsweise 500 µg in einem Experiment aufgeschlossen und analysiert werden. Somit wird das Ergebnis repräsentativer und unterliegt keinen Fehlern, die aus der Heterogenität der Probe resultieren, da bei der Messung die gesamte Probe verwertet wird. Es können daher zeitaufwendige Wiederholungsmessungen gespart werden.

### Ausführungsbeispiel:

Der Aufschluß von Silikaten oder opalem Silizium, wie es in den Schalen von bestimmten Organismen, wie Diatomeen, abgelegt wird, ist von besonderem Interesse. Das darin enthaltene Isotopenverhältnis des Sauerstoffes gestattet Hinweise auf die Umgebungstemperatur zur Zeit der Bildung dieser Siliziumverbindungen zu ziehen. Dabei ist allerdings eine Vorbehandlung des Materials zur Entfernung von angelagerten Wassermolukülen und eingeschlossenen OH-Gruppen notwendig.
In dem beispielhaft dargestellten Verfahren wird die Vorbehandlung in einem Arbeitsgang mit der Freisetzung des Sauerstoffes und dessen Umwandlung in CO oder CO₂ durchgeführt. Das zu untersuchende Material wird in die Graphitküvette eingebracht und in das Gehäuse 5, das als Hochtemperaturzelle fungiert, eingesetzt. Nach Evakuierung dieser Hochtemperaturzelle auf p ≈ 10⁻⁶bar, erfolgt die langsame induktive Aufheizung der Graphitküvette 1. Dabei wird zunächst das adsorbierte Wasser desorbiert und abgepumpt (T ≤ 120°C). Mit steigenden Temperaturen werden die eingelagerten OH-Gruppen entfernt. Nach Beendigung dieses Vorgangs erfolgt die Aufheizung des Materials auf 2000 bis 2200°C, wodurch sich das SiO₂ zersetzt und der Sauerstoff mit dem Graphit der Graphitküvette 1 zu CO₂ und oder CO reagiert. Aufgrund des Bouduard-Gleichgewichts liegt bei Temperaturen um 2000°C praktisch nur CO im Gasraum vor. Dieses kann nach Heizungsende online mittels Helium als Trägergas in den Analysator eines Isotopen-Massenspektrometers überführt werden.
Alternativ kann statt CO auch CO₂ massensprektrometrisch analysiert werden. Dazu wird nach Ende der Aufschlußzeit die Graphitküvette 1 langsam abgekühlt, wodurch sich das Bouduard-Gleichgewicht sukzessive auf die Seite des CO₂ verschiebt. Nach Beendigung des Abkühlvorgangs kann CO₂ analog zu CO mit Hilfe von Helium als Trägergas online zur Bestimmung des Sauerstoffisotopenverhältnisses gemessen werden (Flowmode).
Um eine Vorstellung über das Sauerstoffisotopenverhältnis der angelagerten Wassermoleküle zu erhalten, kann das desorbierte Wasser auch in Kühlfinger 7 einkondensiert werden. Dies kann nach Beendigung des eigentlichen Experiments, oder im Fall von Wasser, direkt nach Verdampfen aus dem Kühlfinger 7 heraus bei gelbglühender Graphitkküvette 1 (ca. 1100°C) in CO und H umgesetzt werden. Der Wasserstoff wird in dem Palladiumring 14 gelöst, bzw. von diesem aufgenommen und das CO kann anschließend online massenspektroskopisch auf seine Sauerstoffisotope hin analysiert werden. OH-Gruppen lassen sich ebenfalls an heißem Graphit spalten, wobei CO und Wasserstoff entsteht. Das Vorgehen ist analog dem Prozeß zu Wasser. Die Hochtemperaturzelle ist so konzipiert, daß über einen speziellen Probenteller automatisch eine größere Anzahl von Proben abgearbeitet werden kann. Massenspektrometer und Hochtemperaturzelleneinheit sind so zusammenfaßbar, daß on-line im Flow-Mode gemessen werden kann.

## Patentansprüche

1. Verfahren zur Freisetzung von Sauerstoffisotopen aus Silikaten,
**dadurch gekennzeichnet,**
**dass** die Silikate mit hochreinem Graphitpulver oder einer Graphitküvette (1) aus hochreinem Graphit, welche Mittel zum Ablösen von Verunreinigungs- und Fremdpartikeln aufweist, in Kontakt gebracht und sequentiell mittels Induktion in einem vakuumdichten Gehäuse (5) aus Quarzglas, welches mit Hilfe von Kühlmitteln gekühlt wird, unter Austreibung von Verunreinigungen wie Wasser, OH-Gruppen und/oder stickstoffhaltigen Verbindungen aufgeheizt werden, wobei CO und/oder CO₂ entsteht.

2. Verfahren nach Anspruch 1,
**dadurch gekennzeichnet,**
**dass** das bei der Aufheizung des Silikates entstehende CO oder CO₂ isoliert wird.

3. Verfahren nach einem der Ansprüche 1 bis 2,
**dadurch gekennzeichnet,**
**dass** das CO oder CO₂ einem Analysenverfahren zugeführt wird.

4. Verfahren nach Anspruch 3,
**dadurch gekennzeichnet,**
**dass** das Analysenverfahren ein isotopenmassenspektroskopisches Verfahren ist.

5. Verfahren nach einem der Ansprüche 1 bis 4,
**dadurch gekennzeichnet,**
**dass** eine Aufheizung auf 1600 bis 2200 °C vorgenommen wird.

6. Vorrichtung zur Ausführung des Verfahrens nach Anspruch 1,
**dadurch gekennzeichnet,**
**dass** sie eine Graphitküvette (1) mit Bohrungen (11 a,b) besitzt, welche in radialer Richtung von einer axialen Bohrung (3) ausgehen, und eine Induktionsquelle umfaßt, dass sich die Graphitküvette (1) in einem vakuumdichten Gehäuse (5) aus Quarzglas befindet, an welchem eine Pumpe angeschlossen ist, dass sie Mittel (7) zum Auffangen von durch die Induktion entstandenem gasförmigem CO oder CO₂ besitzt, dass das Gehäuse (5) aus Quarzglas mit Mitteln zum Kühlen (8) versehen ist.

7. Vorrichtung nach Anspruch 6,
**dadurch gekennzeichnet,**
**dass** das Gehäuse (5) aus Quarzglas bodenseitig zu öffnen ist, damit der Feststoff mit der Graphitküvette gewechselt werden kann.

8. Vorrichtung nach Anspruch 6 oder 7,
**dadurch gekennzeichnet,**
**dass** die Graphitküvette (1) längsförmig ausgebildet ist, wobei sich am oberen Ende eine Aushöhlung (2) für die Aufnahme des Feststoffes befindet und am entgegengesetzten Ende eine axiale Bohrung (3) vorhanden ist, welche einen Stab aufnehmen kann, mit dem die Graphitküvette in das Gehäuse (5) eingeführt werden kann.

## Revendications

1. Procédé pour libérer des isotopes de l'oxygène de silicates,
**caractérisé**
**en ce que** l'on met les silicates en contact avec de la poudre de graphique de grande pureté ou avec une cuvette (1) de graphique en graphique de grande pureté qui a des moyens de détachement de particules d'impuretés et étrangères et on les chauffe séquentiellement au moyen d'une induction dans une enceinte (5) étanche au vide en verre de quartz, qui est refroidie à l'aide de moyens de refroidissement avec extraction des impuretés, comme de l'eau, des groupes OH et/ou des composés azotés, avec formation de CO et/ou de CO₂.

2. Procédé suivant la revendication 1;
**caractérisé,**
**en ce que** l'on isole du CO ou du CO₂ qui se forme lors de chauffage du silicate.

3. Procédé suivant l'une des revendications 1 à 2,
**caractérisé en ce que** l'on envoie le CO ou le CO₂ à un procédé d'analyse.

4. Procédé suivant la revendication 3,
**caractérisé en ce que** le procédé d'analyse est un procédé de spectroscopie de masse d'isotopes.

5. Procédé suivant l'une des revendications 1 à 4,
**caractérisé en ce que** l'on effectue un chauffage jusqu à 1 600 à 2 200° C.

6. Dispositif de mise en oeuvre du procédé suivant la revendication 1;
**caractérisé,**
**en ce qu'**il comprend une cuvette (1) en graphique ayant des trous (11 a, b) qui partent en direction radiale d'un trou (3) axial et une source d'induction, en ce que la cuvette (1) en graphique se trouve dans une enceinte (5) étanche au gaz en verre de quartz à laquelle est raccordée une pompe en ce qu'il a des moyens (7) de capture de CO ou CO₂ gazeux formé par l'induction, en ce que l'enceinte (5) en verre de quartz est muni de moyens de refroidissement (8).

7. Dispositif suivant la revendication 6,
**caractérisé en ce que** l'enceinte (5) en verre de quartz est ouverte du côté du fond afin de pouvoir remplacer la matière solide par la cuvette en graphique.

8. Dispositif suivant la revendication 6 ou 7,
**caractérisé en ce que** la cuvette (1) en graphique est longiforme, une cavité (2) de réception de la matière solide se trouvant à l'extrémité supérieure et un trou (3) axial étant prévu à l'extrémité opposée, trou qui peut recevoir un barreau par lequel la cuvette en graphique peut être introduite dans l'enceinte (5).

## Claims

1. A method of liberating oxygen isotopes from silicates,
**characterised in that**
the silicates are placed in contact with high-purity graphite powder or with a graphite vessel (1) made of high-purity graphite, which comprise means for removing impurity particles and extraneous particles, and are heated sequentially by means of induction in a vacuum-tight housing (5) made of quartz glass, which is cooled by means of coolants, with the expulsion of impurities such as water, OH groups and/or nitrogen-containing compounds, whereupon CO and/or CO₂ are formed.

2. A method according to claim 1,
**characterised in that**
the CO or CO₂ which is formed when the silicate is heated is isolated.

3. A method according to either one of claims 1 or 2,
**characterised in that**
the CO or CO₂ is supplied to a method of analysis.

4. A method according to claim 3,
**characterised in that**
the method of analysis is an isotope mass spectrometer method.

5. A method according to any one of claims 1 to 4,
**characterised in that**
heating is effected to 1600 to 2200°C.

6. An apparatus for carrying out the method according to claim 1,
**characterised in that**
it comprises a graphite vessel (1) with holes (11 a, b) which lead off radially from an axial bore (3), and comprises an induction source, that the graphite vessel (1) is situated in a vacuum-tight housing (5) made of quartz glass to which a pump is connected, that it has means (7) for capturing gaseous CO or CO₂ formed due to said induction, and that the housing (5) made of quartz glass is provided with means for cooling (8).

7. An apparatus according to claim 6,
**characterised in that**
the housing (5) made of quartz glass can be opened at its base so that the solid with the graphite vessel can be changed.

8. An apparatus according to claim 6 or 7,
**characterised in that**
the graphite vessel (1) is of elongated construction, wherein a recess (2) for receiving the solid is situated at the top end, and an axial bore (3), which can receive a rod with which the graphite vessel can be introduced into the housing (5), is present at the opposite end.
